Europäisches Patentamt

European Patent Office

Office européen des brevets

(11) Veröffentlichungsnummer: **0 280 229**
**A2**

(19)

(12) ## EUROPÄISCHE PATENTANMELDUNG

(21) Anmeldenummer: 88102563.9

(22) Anmeldetag: 22.02.88

(51) Int. Cl.4: **G01N 25/56 , G01N 27/18**

(30) Priorität: 27.02.87 DE 3706501

(43) Veröffentlichungstag der Anmeldung:
31.08.88 Patentblatt 88/35

(84) Benannte Vertragsstaaten:
CH DE FR GB IT LI

(71) Anmelder: **LUCAS INDUSTRIES public limited company**
**Great King Street**
**Birmingham, B19 2XF West Midlands(GB)**

(72) Erfinder: **Vath, Josef**
**Kurfürst-Schönborn-Strasse 124**
**D-5400 Koblenz(DE)**
Erfinder: **Schmitt, Hubert Erich**
**Mohlenweg 40**
**D-5405 Ochtendung(DE)**
Erfinder: **Hobein, Dirk**
**Am Kalmen 4**
**D-5400 Koblenz(DE)**
Erfinder: **Miessen, Hermann-Josef**
**Blumenstrasse 8**
**D-5400 Koblenz(DE)**
Erfinder: **Benz, Theodor**
**Sankt-Bernhard-Strasse 124**
**D-5400 Koblenz(DE)**

(74) Vertreter: **von Hellfeld, Axel, Dipl.-Phys. Dr. et al**
**Patentanwälte Wuesthoff- v. Pechmann-Beherens-Goetz**
**Schweigerstrasse 2**
**D-8000 München 90(DE)**

(54) Verfahren zum Ermitteln des Wassergehaltes einer hydraulischen Flüssigkeit.

(57) Zum Ermitteln des Wassergehaltes einer Bremsflüssigkeit wird ein elektrisch geheizter, temperaturabhängiger Widerstand in die Bremsflüssigkeit eingetaucht und der durch den Widerstand fließende Strom wird kontinuierlich erhöht, wobei sich die Temperatur des Widerstands monoton bis zu einem ersten Maximum I erhöht und dann zumindest ein erstes Minimum A durchläuft. Der Temperaturverlauf des Widerstandes ab dem ersten Maximum wird zur Ermittlung des Wassergehaltes der Bremsflüssigkeit herangezogen.

FIG.1

## Verfahren zum Ermitteln des Wassergehaltes einer hydraulischen Flüssigkeit

Die Erfindung betrifft ein Verfahren zum Ermitteln des Wassergehaltes einer hydraulischen Flüssigkeit, wie Bremsflüssigkeit, mittels eines in die Flüssigkeit eingetauchten, elektrisch beheizten, temperaturabhängigen Widerstandes, dessen Temperatur-und Widerstandswertänderung in Abhängigkeit von der Zeit gemessen wird.

Ein derartiges Verfahren ist aus der europäischen Patentschrift 74415 bekannt.

In Hydraulikanlagen verwendete Flüssigkeiten ("hydraulische Flüssigkeiten" genannt) auf der Basis von Polyglykolether sind hydrophil, können also Wasser aufnehmen. Insbesondere bei Bremsflüssigkeiten in Fahrzeugen kann die Aufnahme von Wasser nachteilige Folgen haben. Darüberhinaus sind die in Rede stehenden Bremsflüssigkeiten auch hygroskopisch, ziehen also Wasser an.

Hydraulische Bremsflüssigkeiten auf der Basis von Polyglykolen und Polyglykolethern nehmen deshalb mit zunehmender Betriebszeit Wasser bis zum Sättigungsgrad auf. Mit steigendem Wassergehalt sinkt aber der Siedepunkt der Bremsflüssigkeit erheblich ab, und zwar typischerweise von einem Siedepunkt bei 280°C bei wasserfreier Bremsflüssigkeit bis auf einen Siedepunkt von etwa 150°C bei einem Wassergehalt von ca. 5 %.

In hydrophoben Flüssigkeiten hingegen, wie Härteöl, ist Wasser höchstens emuligiert, es bleiben also kleine Wassertröpfchen in ihm bestehen. Demgegenüber wird in hydrophilen Flüssigkeiten Wasser gelöst; das Wasser geht also eine chemische Bindung mit der Bremsflüssigkeit ein.

Fahrzeugbremsen können bei intensiver oder häufiger Betätigung, wie sie bei Vollbremsungen oder häufigem Bremsen auf längeren Gefällestrecken, wie Paßstraßen im Gebirge, auftreten, sehr hohe Temperaturen erreichen. Enthält die Bremsflüssigkeit zu viel Wasser, so bilden sich bei Erhitzung der Bremse Dampfblasen in der Bremsflüssigkeit, wodurch die Funktionsfähigkeit der Bremse beeinträchtigt werden kann. Die Dampfblasen sind namlich kompressibel, so daß der am Bremspedal erzeugte Druck in der Hydraulikflüssigkeit schlimmstenfalls nur zu einer Kompression der Dampfblasen führt, ohne daß die Bremse anspricht.

Es ist deshalb wünschenswert, die Bremsflüssigkeit auf ihren Wassergehalt, d.h. auf die Temperatur überprüfen zu können, bei der sich Dampfblasen bilden.

In der EP 74415 wird ein Verfahren zum Ermitteln der Siedetemperatur von hygroskopischen Flüssigkeiten vorgeschlagen, bei dem ein in die Flüssigkeit eingetauchtes Heizelement elektrisch derart aufgeheizt wird, daß eine örtliche Verdampfung der Flüssigkeit erfolgt, wobei sichergestellt ist, daß die sich bildenden Dampfblasen vom Heizelement abgeführt werden, und die sich infolge der Dampfblasenbildung einstellende konstante Temperatur des Heizelements von einem Temperatur-Meßelement gemessen wird und als Maß für den Siedepunkt der Flüssigkeit dient. Bei dem bekannten Verfahren wird eine vorgegebene Spannung ohne zeitlichen Anstieg direkt an den Sensor-Widerstand angelegt und es soll sich dann in Abhängigkeit vom Siedepunkt der Flüssigkeit eine konstante Temperatur des Widerstandes einstellen, d.h. der Strom durch den Sensor-Widerstand soll nach einer Anstiegsphase einen konstanten Wert erreichen.

Praktische Versuche mit dem bekannten Verfahren haben aber gezeigt, daß nach Anlegen einer Spannung an den Sensor-Widerstand nach sehr kurzer Zeit ein konstanter Strompegel erreicht wird, der unabhängig davon ist, ob die Flüssigkeit siedet oder nicht. Es wurden für eine Vielzahl von Sensor-Spannungen jeweils die sich nach kurzer Zeit einstellenden Ströme und Spannungen am Widerstandssensor gemessen, ohne daß der sich einstellende Strom-bzw. Spannungspegel einen Rückschluß auf den Siedepunkt und damit den Wassergehalt der Flüssigkeit erlaubte. Mit einer derartigen Messung ist der Wassergehalt der Flüssigkeit also nicht zuverlässig zu ermitteln.

Es wird angenommen, daß die Schwierigkeiten, bei den bekannten Verfahren zuverlässige Meßergebnisse zu erzielen, darauf beruhen, daß der Wärmeübergang vom Widerstandssensor in die zu prüfende Flüssigkeit von einer Vielzahl von Parametern abhängt, die kaum zuverlässig zu steuern sind. Am Siedepunkt der Flüssigkeit wird durch die aufsteigenden Dampfblasen eine Strömung erzeugt, durch die kontinuierlich frische Flüssigkeit an den Widerstandssensor geführt wird, so daß ein erhöter Wärmeübergang vom Sensor in die Flüssigkeit stattfindet.

Andererseits ist aber bekannt, daß der Wärmeübergang von einer Festkörperoberfläche in eine Flüssigkeit eine sehr empfindliche Funktion insbesondere des Bewegungszustandes der Flüssigkeit ist. Der Wärmeübergang von der Oberfläche des Widerstandssensors in die zu prüfende Flüssigkeit ergibt sich in ersten Näherung aus folgender Gleichung:

$$Q = \alpha \ F \ (T_s - T_f)$$

wobei:

Q: die vom Sensor in die Flüssigkeit pro Zeiteinheit abgegebene Wärme;

$\alpha$: die Wärmeübergangszahl;

F: die Größe der wärmeabgebenden Oberfläche des Sensors; und

$T_s$-$T_f$: die Temperaturdifferenz zwischen der wärmeabgebenden Sensoroberfläche und der diese Fläche benetzenden Flüssigkeit ist.

Es ist bekannt ("Kraftfahrttechnisches Taschenbuch" der Firma BOSCH, 19. Aufl., S. 85), daß die Wärmeübergangszahl empfindlich von den Strömungsverhältnissen abhängt. Beispielsweise beträgt die Wärmeübergangszahl $\alpha$(gemessen in W/m$^2$ $^\times$ K) bei Wasser an einer ebenen Wand 500 bis 2000, während bei bewegtem Wasser Wärmeübergangszahlen von 2000 bis 4000 und bei siedendem Wasser Werte von 2000 bis 6000 gemessen werden. Somit kann die Wärmeübergangszahl zwischen einem Festkörper und einer Flüssigkeit um einen Faktor 10 in Abhängigkeit vom Zustand der Flüssigkeit differieren. Diese Schwankungsbreite der Wärmeübergangszahl bewirkt, daß auch die sich am Widerstandssensor einstellende Temperatur entsprechenden Schwankungen unterliegt. Bei einer Bremsflüssigkeit kommt hinzu, daß deren Strömungsverhalten stark von der temperaturabhängigen Viskosität abhängt, wodurch eine weitere Unsicherheit bezüglich des Wärmeüberganges zwischen Widerstandssensor und Flüssigkeit bedingt ist.

Aus der GB 20 42 737 ist ein Verfahren bekannt, mit dem hydrophobe Flüssigkeiten auf ihren Wassergehalt untersucht werden. In diesen Flüssigkeiten wird, wie oben gesagt, im Unterschied zu hydrophilen Flüssigkeiten das Wasser nicht gelöst, sondern bleibt in Form kleiner Wassertröpfchen bestehen. Während also bei einem hydrophoben Härteöl bei einer Erhitzung auf z.B. 200°C sich solange Dampfblasen bilden, wie überhaupt Wasser in der Flüssigkeit enthalten ist, bilden sich bei einer hydrophilen Bremsflüssigkeit bei einer Erhitzung auf z.B. 200°C nur dann Dampfblasen, wenn der Anteil des Wassers an der Bremsflüssigkeit einen bestimmten Wert, z.B. 2 %, überschreitet. Bei dem Verfahren gemäß der GB 20 42 737 wird ein temperaturabhängiger Widerstand in das Öl-Bad eingetaucht und mit elektrischem Strom beschickt. Der Strom wird so geregelt, daß der Widerstand eine konstante Temperatur aufweist, die oberhalb des Siedepunktes von Wasser liegt, also z.B. auf 110°C. Sobald Wassertröpfchen in Öl in die Gasphase übergehen (Verdampfen), kommt es am _temperaturabhängigen Widerstand zu einem Temperatur-Abfall. Dadurch ändert sich der Widerstandswert und eine Brücke wird verstimmt. Der Stromfluß durch den Widerstand wird dann so geregelt, daß die konstante Temperatur aufrecht erhalten bleibt. Als Meßgröße dient die Zahl der Stromstöße, die zur Nachregelung der Widerstandstemperatur erforderlich sind. Die Anzahl der Stromstöße ist eine Funktion der Anzahl der in der Flüssigkeit vorhandenen Wasser-Tröpfchen.

Die pro Zeiteinheit gemessenen Stromstöße erlauben also eine Aussage über die Menge des im Härteöl enthaltenen Wassers sowie über die Brauchbarkeit des Öls. Bei dem bekannten Verfahren wird ein Schwellenwert bezüglich der Anzahl der Stromstöße pro Zeiteinheit vorgegeben. Bei Überschreiten dieses Schwellenwertes wird ein Alarm ausgelöst. Das bekannte Verfahren ermittelt somit nicht kontinuierlich einen Meßwert bezüglich des Wassergehaltes, sondern entscheidet nur, ob ein vorgegebener Schwellenwert unter-oder überschritten wird.

Der Erfindung liegt die Aufgabe zugrunde, ein Verfahren anzugeben, mit dem die Temperatur der Dampfblasenbildung einer hydraulischen Flüssigkeit und damit deren Wassergehalt genau ermittelbar ist.

Erfindungsgemäß wird diese Aufgabe mittels eines in die Flüssigkeit eingetauchten, elektrisch geheizten, temperaturabhängigen Widerstandes dadurch gelöst, daß der Strom durch den und/oder die Spannung am Widerstand kontinuierlich erhöht wird, wobei dessen Temperatur monoton bis zu einem ersten Maximum ansteigt und dann zumindest ein erstes Minimum durchläuft, und daß der Temperaturverlauf des Widerstandes ab dem ersten Maximum mit abgespeicherten Daten verglichen wird.

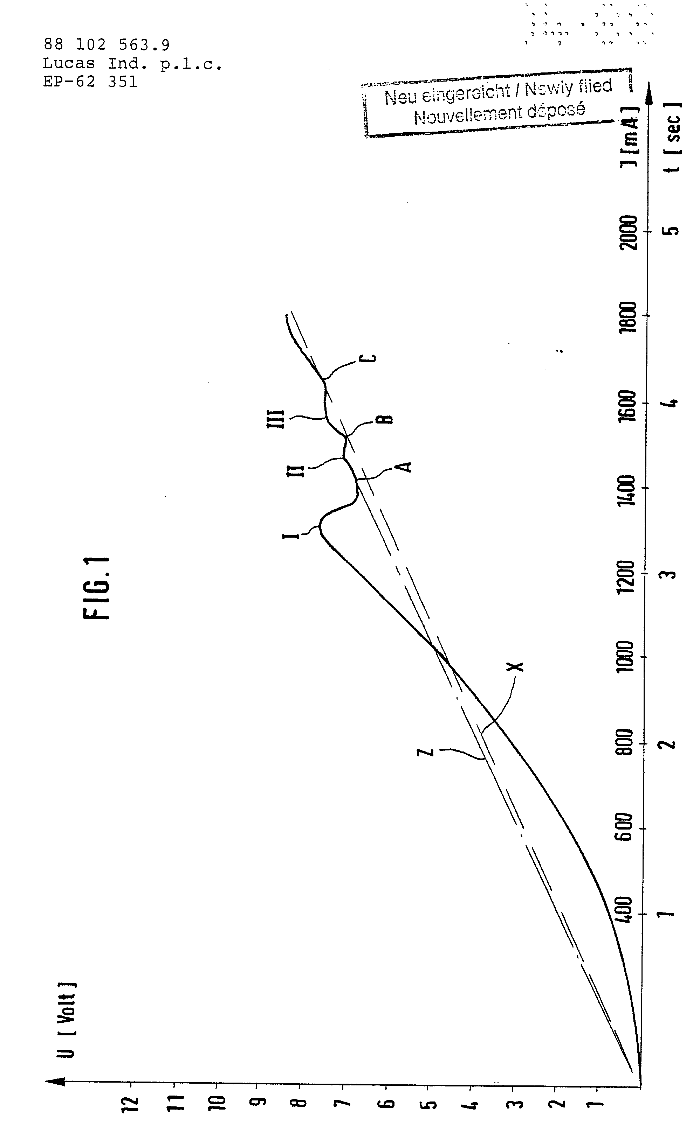

Gemäß der Erfindung wird also an den Sensorwiderstand nicht eine fest vorgegebene, konstante Spannung angelegt, sondern es wird entweder der Strom oder die Spannung am Widerstand derart gesteuert, daß mit der Zeit ein kontinuierlicher Anstieg der gesteuerten Größe erfolgt. In einer bevorzugten Ausgestaltung der Erfindung ist vorgesehen, daß der Widerstand einen positiven Temperatur-Koeffizienten aufweist und daß der Strom durch den Widerstand mit einer konstanten Anstiegsrate erhöht wird. Dabei steigt die über dem Widerstand abfallende Spannung zunächst ebenfalls kontinuierlich an bis ein Maximum erreicht ist (s. Fig. 1). Der Quotient aus Spannung und Strom ergibt den Widerstandswert und ist ein direktes Maß für die Temperatur des Widerstandes. Es wurde überraschend festgestellt, daß nach Erreichen des Maximums die Spannung am Widerstand trotz des kontinuierlich weitersteigenden Stromes kurzzeitig abfällt (d.h. der Widerstand kühlt sich kurzfristig wieder ab), um dann wieder anzusteigen (s. Fig. 1).

Im Anschluß an das erste Maximum steigt die über dem Sensorwiderstand abfallende Spannung nicht so gleichmäßig an, wie vor Erreichen des ersten Maximums. Es treten weitere, allerdings weniger ausgeprägte Maxima und Minima im Strom/Spannungsverlauf auf.

Der Erfindung liegt weiter die Erkenntnis zugrunde, daß die Höhe des ersten Maximums in bezug auf das sich anschließende Minimum für eine gegebene Bremsflüssigkeitsart eine Funktion des Wassergehalts der Flüssigkeit ist. Der Abstand zwischen dem ersten Maximum und dem ersten Minimum ist umso größer, je mehr Wasser in der Flüssigkeit enthalten ist.

Gemäß der Erfindung werden die vorstehend genannten Erkenntnisse derart zur Bestimmung der Temperatur der Dampfblasenbildung in einer hydraulischen Flüssigkeit ausgenutzt, daß der Temperaturverlauf des Widerstandes ab dem ersten Maximum mit abgespeicherten Daten verglichen wird. Für den Vergleich ergeben sich mehrere Möglichkeiten.

Es kann der Abstand zwischen dem ersten Maximum und dem ersten Minimum des Temperaturverlaufs des Widerstandes ermittelt und mit einem Sollwert verglichen werden. Die Differenz zwischen dem ermittelten Wert und dem Sollwert kann als Maß für den Wassergehalt der Flüssigkeit, genauer die Temperatur der Dampfblasenbildung, welche eine Funktion des Wassergehaltes ist, herangezogen werden.

In einer bevorzugten Ausgestaltung der Erfindung ist vorgesehen, daß der Quotient aus Spannung und Strom des Widerstandes für mehrere Meßpunkte des Temperaturverlaufes nach dem ersten Minimum (also desjenigen Minimums, welches sich an das erste Maximum anschließt) gebildet wird und daß der Mittelwert dieser Quotienten mit einem Soll-Wert verglichen wird, wobei die Abweichung ein Maß für die Temperatur ist, bei der sich in der zu prüfenden Flüssigkeit Dampfblasen bilden.

Es wurde überraschend gefunden, daß eine besonders gut auswertbare Meßkurve dann entsteht, wenn der Meßwiderstand mit einer Stromanstiegsgeschwindigkeit von 400 mA/s beaufschlagt wird. Dann entstehen sehr charakteristische Kurvenverläufe mit ausgeprägten Maxima und Minima. Die Zeitdauer des Stromanstiegs mit einem Gradienten von 400 mA/s wird bevorzugt auf 4 s eingestellt. Mit diesen Daten werden gut reproduzierbare Ergebnisse erzielt.

Nachfolgend wird ein Ausführungsbeispiel der Erfindung anhand der Zeichnung näher beschrieben. Es zeigt:

Fig. 1 eine Spannungs/Strom-Kurve für einen Widerstand, durch den ein mit der Zeit kontinuierlich anwachsender Strom fließt; und

Fig. 2 ein Block-Schaltbild einer Vorrichtung zur Durchführung eines Verfahrens zum Ermitteln des Wassergehalts einer Flüssigkeit.

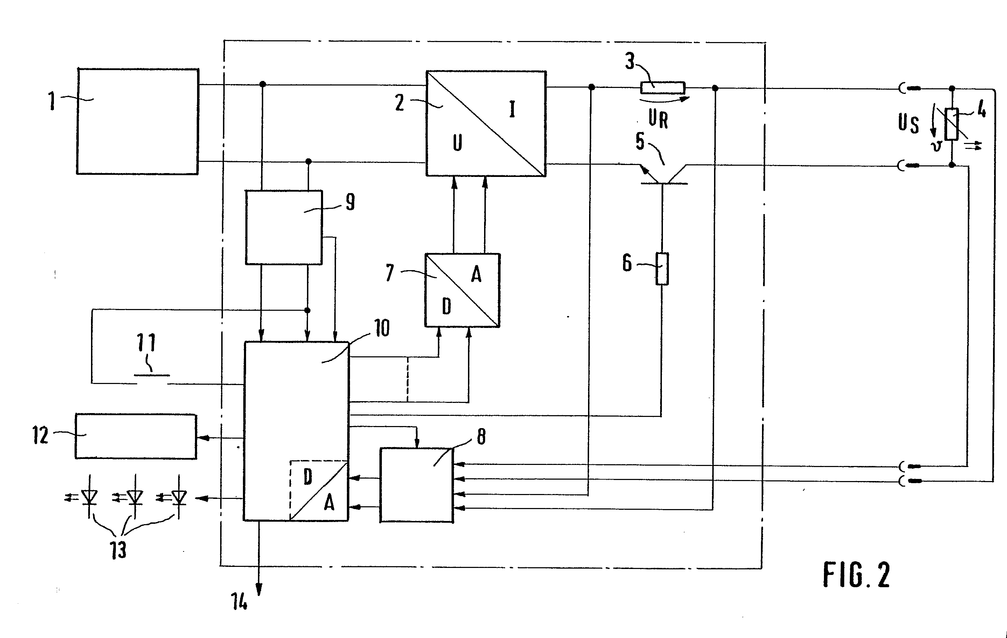

Ein dünner Wolframdraht mit einem Kaltwiderstand von z.B. 2 bis 3 Ohm wird in die zu prüfende Flüssigkeit vollständig eingetaucht und mittels der in Fig. 2 gezeigten, weiter unten näher beschriebenen Schaltung wird ein mit konstanter Anstiegsrate wachsender Strom durch den Widerstandsdraht geschickt. In Fig. 1 sind der durch den Widerstand fließende Strom und die Zeit auf der Abszisse aufgetragen. Es wird die über dem Widerstand abfallende Spannung in Abhängigkeit vom Strom gemessen. Die gemessene Spannung ist auf der Ordinate aufgetragen.

Gemäß Fig. 1 weist die Spannungs/Strom-Kurve zunächst einen gleichmäßigen Anstieg mit wachsendem Gradienten auf, bis ein erstes Spannungsmaximum I erreicht ist. Mit weiter kontinuierlich steigendem Strom fällt die Spannung am Widerstand nach Durchlaufen des ersten Maximums auf ein erstes Minimum A ab. Danach steigt die Spannung wieder an und durchläuft weitere Maxima II, III ... und dazwischenliegende Minima B, C ...

Der in Fig. 1 schematisch gezeigte Strom/Spannungs-Verlauf ist typisch für eine synthetische Bremsflüssigkeit auf der Basis von Polyglykolen oder Polyglykoläther, mit relativ hohem Wassergehalt und dementsprechend niedriger Temperatur der Dampfblasenbildung.

Wird die gleiche Bremsflüssigkeit mit geringerem Wassergehalt verwendet, so ist das erste Maximum I in bezug auf das erste Minimum A weniger ausgeprägt als in Fig. 1 gezeigt. Diese Änderung des Kurvenverlaufes in Abhängigkeit vom Wassergehalt der Flüssigkeit läßt sich dadurch zur Bestimmung der Temperatur der Dampfblasenbildung ausnutzen, daß die mit der zu prüfenden Flüssigkeit gemessene Kurve mit zuvor empirisch ermittelten Soll-Kurven verglichen wird. Die Abweichung der beiden Kurven voneinander ist ein Maß für den Wassergehalt der Flüssigkeit. Für den Vergleich können unterschiedliche Kurven-Daten herangezogen werden.

Insbesondere ist der Mittelwert aus den sich an das erste Spannungsminimum A anschließenden Widerstandswerten ein brauchbarer Wert für die Beurteilung des Wassergehalts der Flüssigkeit.

Der Fig. 1 ist zu entnehmen, daß der Quotient U/I (also der Widerstandswert des Widerstands bzw. dessen Temperatur) am ersten Minimum A, welcher durch die Steigungsgerade Z repräsentiert wird, größer ist als der Mittelwert der sich an das erste Minimum A anschließenden Minima B, C usw., welcher durch die Steigungsgerade X gegeben ist. Auch die Steigung der Geraden X, also der Mittelwert der sich an das erste Minimum A anschließenden Quotienten $U/I$ an jeweils den Stellen der weiteren Minima B, C ... ist ein brauchbares Maß für den Wassergehalt der Flüssigkeit.

Beim gezeigten Ausführungsbeispiel beträgt die Stromanstiegsrate 400 mA/sec. Die gesamte Messung dauert ca. 4 Sekunden. Experimentell wurde ermittelt, daß eine Gesamt-Meßdauer von 3 bis 6 Sekunden gute Ergebnisse liefert. Die Stromanstiegsraten sollten im Bereich von 200 bis 600 mA/sec liegen.

Als Widerstand sind Wolframdrähte gut geeignet. Es ist auch möglich, den Widerstand derart auszubilden, daß auf einem Keramiksubstrat eine dünne Metallschicht, z.B. Wolfram, aufgetragen wird.

Fig. 2 zeigt ein Blockdiagramm einer Schaltung, mit welcher der Widerstand bestromt wird und die Messungen ausgeführt werden.

Die Schaltung besteht aus einer Spannungsquelle 1, einer nachgeschalteten Stromquelle 2, einem Vorwiderstand 3 (dieser Widerstand ist in Reihe mit dem in die zu prüfende Flüssigkeit eingetauchten Widerstand geschaltet), einem Meßwiderstand 4 (hierbei handelt es sich um den in die zu prüfende Flüssigkeit eingetauchten Widerstand aus z.B. Wolfram oder dergleichen), einem Abschalttransistor 5, einem Basiswiderstand 6, einem Digital/ Analog-Wandler 7, einem Multiplexer 8, einem Spannungsregler 9, einem Mikroprozessor 10 (einschließlich eines Analog/Digital-Wandlers), einer Starttaste 11, einer Anzeigeeinrichtung 12, Leuchtdioden 13 und einer seriellen Schnittstelle 14.

Die über dem Vorwiderstand 3 abfallende Spannung wird gemessen und ist mit $U_R$ bezeichnet. Die über dem in die Prüfflüssigkeit eingetauchten Meßwiderstand 4 abfallende Spannung wird ebenfalls gemessen und ist mit $U_S$ bezeichnet.

Die Funktion der in Fig. 2 gezeigten Schaltung ist wie folgt. Das Drücken der Starttaste 11 initiiert im Mikroprozessor 10 das Prüfprogramm. Der Mikroprozessor 10 steuert den Digital/Analog-Wandler 7, welcher die Steuerspannung für die spannungsgesteuerte Stromquelle 2 liefert. Die externe Spannungsquelle 1 versorgt die spannungsgesteuerte Stromquelle 2 und den Spannungsregler 9 für den Mikroprozessor 10. Die Stromquelle 2 ist so zeitlich gesteuert, daß der Strom durch den Meßwiderstand 4 mit einer Anstiegsrate von 400 mA/sec kontinuierlich ansteigt. Der Meßwiderstand 4 hat einen Kaltwiderstandswert von 2,5 Ohm, während der Vorwiderstand 3 einen Widerstandswert von 1 Ohm hat.

Die am Vorwiderstand 3 abfallende Spannung $U_R$ wird dem Multiplexer 8 zugeführt, ebenso die am Meßwiderstand 4 abfallende Meßspannung $U_S$.

Der Multiplexer 8 führt alle 20 μsec abwechselnd dem Mikroprozessor 10 eine dieser beiden Spannungen zu. Um eine genaue Messung zu ermöglichen, wird eine Vier-Leiter-Messung durchgeführt. Der Transistor 5 ist in den Versorgungskreis des Widerstands 4 geschaltet und dient zum schnellen Abschalten des durch die Widerstände fließenden Stromes.

Der Mikroprozessor 10 steuert die Anzeigeeinrichtung 12 entsprechend dem Meßergebnis, also dem Vergleich der gemessenen Kurve entsprechend Fig. 1 mit vorgespeicherten Werten. Gemäß dem Meßergebnis wird mit der Anzeigeeinrichtung 12 ein Wert für den Wassergehalt bzw. den Zustand der Bremsflüssigkeit direkt angezeigt.

Alternativ ist es möglich, mittels der Leuchtdioden 13 in den Farben Rot, Gelb und Grün dem Benutzer anzuzeigen, ob der Wassergehalt der Flüssigkeit schlechter ist als ein vorgegebener Grenzwert, in einem mittleren Bereich liegt bzw. besser ist als ein weiterer vorgegebener Grenzwert.

Die serielle Schnittstelle 14 kann an einen zentralen Computer des Kraftfahrzeuges oder auch an einen Auswerte-Computer der Werkstatt angeschlossen werden, in welcher die Bremsflüssigkeit überprüft wird.

## Ansprüche

1. Verfahren zum Ermitteln des Wassergehaltes einer hydraulischen Flüssigkeit, wie Bremsflüssigkeit, mittels eines in die Flüssigkeit eingetauchten, elektrisch geheizten, temperaturabhängigen Widerstandes, dessen Temperatur- und Widerstandswertänderung in Abhängigkeit von der Zeit gemessen wird,
dadurch **gekennzeichnet**,
daß der Strom durch den und/oder die Spannung am Widerstand kontinuierlich erhöht wird, wobei dessen Temperatur monoton bis zu einem ersten Maximum ansteigt und dann zumindest ein erstes Minimum durchläuft, und daß der Temperaturverlauf des gemessenen Widerstandswertes ab dem ersten Maximum mit abgespeicherten Daten verglichen wird.

2. Verfahren nach Anspruch 1,
dadurch **gekennzeichnet**,
daß der Widerstand einen positiven Temperatur-Koeffizienten aufweist und seine Temperatur aus dem Strom/Spannungs-Verlauf bestimmt wird.

3. Verfahren nach einem der Ansprüche 1 oder 2,
dadurch **gekennzeichnet**,
daß als Widerstand ein Draht verwendet wird.

4. Verfahren nach einem der vorhergehenden Ansprüche,

dadurch **gekennzeichnet,**

daß der Strom durch den Widerstand mit einer konstanten Anstiegsrate erhöht und die an ihm abfallende Spannung gemessen wird.

5. Verfahren nach Anspruch 4,

dadurch **gekennzeichnet,**

daß der Quotient aus Spannung und Strom des Widerstandes für mehrere Meßpunkte der Strom/Spannungs-Kurve des Widerstandes nach dem ersten Minimum gebildet wird und daß der Mittelwert dieser Quotienten mit einem Soll-Wert verglichen wird.

FIG.1

88 102 563.9
Lucas Ind. p.l.c.
EP-62 351

0 280 229

FIG. 2

0 280 229